Europäisches Patentamt

European Patent Office

Office européen des brevets

(19)

(11) Publication number: **0 255 525 B1**

(12) **EUROPEAN PATENT SPECIFICATION**

(45) Date of publication of patent specification: **30.09.92**  (51) Int. Cl.⁵: **A61K 7/06**, A61K 7/32

(21) Application number: **87900619.5**

(22) Date of filing: **31.12.86**

(86) International application number:
**PCT/US86/02833**

(87) International publication number:
**WO 87/04341 (30.07.87 87/17)**

(54) **METHOD FO NEUTRALIZING MALODOR DERIVED FROM AXILLA.**

(30) Priority: **16.01.86 US 819275**

(43) Date of publication of application:
**10.02.88 Bulletin  88/06**

(45) Publication of the grant of the patent:
**30.09.92 Bulletin  92/40**

(84) Designated Contracting States:
**AT BE CH DE FR GB IT LI LU NL SE**

(56) References cited:
US-A- 2 521 713     US-A- 2 893 918
US-A- 3 184 376     US-A- 3 555 145
US-A- 3 655 868     US-A- 3 856 941
US-A- 4 043 932     US-A- 4 261 849
US-A- 4 382 079

**PHARMACEUTICAL FORMULAS, FORRESTER,
May 1954, vol. I; pp. 392, 419, 436, 439, 440,
442, 443**

(73) Proprietor: **THE GILLETTE COMPANY
Prudential Tower Building
Boston Massachusetts 02199(US)**

(72) Inventor: **BLANKENSHIP, Iris, Davis
9103 Turtle Dove Lane
Gaithersburg, MD 20879(US)**

(74) Representative: **Baillie, Iain Cameron et al
c/o Ladas & Parry, Altheimer Eck 2
W-8000 München 2(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 255 525 B1

**Description**

The present invention relates to compounds which neutralize axillary malodor.

Various approaches have been taken to the problem of axillary malodor. One approach has been the use of deodorants which may contain germicides and/or a fragrance. Germicides inhibit the reproduction of the bacteria which are believed to contribute to the production of axillary malodor. Fragrances are used to mask any odor produced. Another approach to the prevention of malodor is the use of antiperspirants. Antiperspirants inhibit bacteria and reduce the amount of sweat production thereby limiting the formation of substrate which gives rise to axillary malodor.

Certain metals and metal salts have been suggested for use in the control of odors. For example, Japanese Disclosure No. 83-222011 describes a cream for removing odor from the axilla which includes copper powder and perfume in a base of cosmetic cream. The disclosure postulates that the copper powder acts on the secretion of the odor, thereby suppressing generation of the malodor.

Another description of the use of metal salts against odor appears in French Patent No. 1,394,875. This French patent describes the use of water-soluble iron and copper compounds to reduce or eliminate body odors. Various salts and complexes of cupric copper and ferrous iron are mentioned, including sulfates, chlorides, acetates, gluconates, citrates, tartrates, salts of ethylenediamine tetraacetic acid sodium or potassium, and iron and copper chlorophyllins. Various iron and copper phosphates are also mentioned, especially for use as food additives. The most effective combinations are indicated to be those in which both metals are present in the ionizable and nonionizable forms. The only testing reported in the French patent was odor reduction of sewage sludge, and odor reduction in mice by measurement of the odor of the entire mouse, fecal odor and urine odor.

German Patent No. 1,083,503 describes the deodorizing action of metal complexes of 1,3-diketones. Listed metals include copper, nickel, cobalt, calcium, zirconium, zinc, tin, aluminum, cadmium, cerium, beryllium, magnesium, and mercury. Their use in various ways, including topical application to the axilla, is discussed. These compounds are theorized to function by interfering with the metabolism of the odor-producing microorganisms.

British Patent Application No. 1,581,586 describes a sanitary foot wear article which includes a composition comprising copper, silver, or a copper-silver alloy powder dispersed in and held by water-insoluble resin binder. The British patent application indicates a belief that the metal reacts with substances secreted by the foot to produce metal salts which act as astringents and also act to prevent the growth of microorganisms.

Despite the above, attempts have continued to find compositions which are effective against axillary malodor even after it has been formed.

The present invention is a method of neutralizing axillary malodor by the use of cupric sulfate.

According to the present invention there is provided a method of neutralizing malodor derived from the axilla of a subject, characterized by contacting the malodor by topically applying to the axilla of the subject, or by applying to a fabric which was or will be in contact with said malodor, as a neutralizing compound cupric sulfate, in such a malodor - reducing concentration as to provide a cosmetic improvement of the subject by reduction or elimination of malodor associated with the subject.

The present invention includes the surprising discovery that certain compounds effectively neutralize preformed axillary malodor. This should be contrasted with the prior art where the focus was on either preventing formation of the odor, or on masking it after production. This distinction is particularly important in treating axillary malodor build-up in certain fabrics, particularly polyesters.

The mechanism by which the malodor neutralization occurs has yet to be discerned. Moreover, it appears that more than one mechanism may be involved.

As a part of the attempt to identify the malodor compound, numerous compounds have been tested to determine their ability as malodor neutralizers. This has led to the unexpected and surprising discovery that relatively few compounds are effective in neutralizing axillary malodor. Moreover, many compounds which have previously been suggested as deodorants do not act as neutralizers. Compounds which are effective in malodor neutralization are listed in Table I. The compounds indicated have been confirmed subjectively, i.e., by a sniff test described further below.

## TABLE I

CUPRIC SULFATE

CUPRIC SULFATE + SODIUM BICARBONATE

SILVER SULFATE

SILVER SULFATE + SODIUM BICARBONATE

POTASSIUM PERMANGANATE

FERRIC CHLORIDE

SODIUM HYDROXIDE

SILVER PROTEINATE

SODIUM HYPOCHLORITE

COPPER GLUCONATE (VARIABLE RESULTS)

ZINC SULFATE (VARIABLE RESULTS)

Cupric sulfate is indicated in Table I to have been used alone and in conjunction with sodium bicarbonate. In each case, neutralization with the metal salt alone resulted in a residual faint fatty acid smell which was removed by the application of sodium bicarbonate. In fact, the most preferred composition used in the present invention is the combination of cupric sulfate and sodium bicarbonate. Although sodium bicarbonate by itself did not neutralize axillary malodor, it does tend to neutralize certain other odors such as fatty acid odors, that may be present in the axillae. Although silver sulfate is even more effective than cupric sulfate, it has the disadvantage of staining the skin upon application.

The indication in Table I that zinc sulfate and copper gluconate give variable results should also be noted. The reason why they are only effective in certain of the tests is not known, but may be due to variations in pH, temperature, reaction time, substrate variability and sample variability.

In contrast to the relatively small number of compounds in Table I, Table II lists twice as many compounds which were found not to be effective in neutralization of axillary malodor.

## TABLE II

ZINC GLUCONATE

SILVADENE

ZINC OXIDE

SODIUM SULFATE

ZIRCONIUM SULFATE

CALCIUM SULFATE

ALUMINUM SULFATE

MAGNESIUM SULFATE

FERROUS SULFATE

FERRIC SULFATE

### TABLE II (CON'T)

ALUMINUM CHLOROHYDRATE

COPPER CHLORIDE

ZINC CARBOLATE

SODIUM COPPER CHLOROPHYLLIN

SODIUM BICARBONATE

HYDROGEN PEROXIDE

BENZOYL PEROXIDE

FERRIC AMMONIUM CITRATE

As can be seen from a comparison of Tables I and II, compounds which the prior art suggests are comparable in odor reduction abilities are not comparable when applied to axilla malodor. For example, ferric chloride is effective, but ferric sulfate and ferric ammonium citrate are not. Similarly, cupric sulfate is effective, but copper chloride is not. This apparent lack of any pattern of effectiveness is further evidence of the unexpected and surprising nature of the present invention.

The compounds of the present invention can be applied to the axilla in any of the various delivery systems well known to those skilled in the art. These include deodorant sticks, microencapsulation in a suitable base, dual dispensers, powders, and aerosols in non-aqueous suspensions. Any of the commonly employed ingredients can be used, provided they have no adverse effect on the active ingredient(s).

The concentration of the malodor neutralization compound can vary over a wide range. Typical concentrations are between about 0.001 and about 100% by weight, preferably between about 0.05 and about 5.0%. In the case of aqueous cupric sulfate, the preferred concentration is about 2% wt/vol.

The concentration of sodium bicarbonate is typically between about 0.01% and about 99.9% by weight, preferably between about 0.1 and about 10%.

As discussed above, one of the problems with prior art methods of dealing with axilla malodor was that no method was available for removing the odor from synthetic fabrics. 100% polyester clothing is particularly prone to irreversible absorption of axillary malodor. Such absorption is also seen in cotton/polyester blends. By "irreversible" is meant that the odor is retained even after repeated laundering with detergent. Moreover, soil-release finished polyester fabric retains malodor even more stubbornly than unfinished polyester.

In particular, cupric sulfate was found to be effective in malodor neutralization when employed at 2.0% by weight based upon the weight of the laundering bath. However, cupric sulfate concentration should be considerably below this concentration when laundering white fabrics to avoid producing a greenish tint.

The compound employed for malodor neutralization in fabrics can either be added separately, as a powder or liquid formulation, or incorporated as an additional ingredient in known laundry detergents. Care should be exercised to ensure that no adverse interaction occurs between the detergent and the malodor neutralization compound, particularly when the detergent includes phosphates.

In order to further illustrate the present invention and the advantages thereof, the following specific examples are given.

### EXAMPLES

The following description sets forth the preparation of malodor samples, and the procedure for identifying malodor neutralizers.

### Preparation of Malodor Samples

Aqueous axillary samples were collected daily from a panel of known malodor substrate producers. These odorless samples were pooled, extracted with hexane to remove lipid material and lyophilized. The dry samples were then inoculated with axillary odor-producing bacteria in 10 mℓ distilled water to yield aqueous solutions of acrid malodor. Aliquots of 1-3 ml aqueous malodor were used for malodor neutralization experiments. The larger sample size was used if the level of malodor observed in the headspace of the sample was low.

Procedure for Identifying Malodor Neutralizers

Three drops of reagent were added to 1-3mℓ aliquots of aqueous malodor in a 4mℓ vial. Approximately 0.5-1mℓ of chloroform was added to the vial. The sample was inverted several times. The two layers were allowed to separate for a few minutes. Aliquots of the chloroform layer were removed and evaluated for odor. Three drops of 10% aqueous sodium bicarbonate were then added to the sample. The sample was inverted several times. After allowing the two layers to separate, the chloroform layer was reevaluated for odor. Controls were prepared in the same manner except that three drops of deionized water were used in place of reagent. This extraction technique resulted in concentration of the malodor in the chloroform layer.

Measurement of Odor

Approximately 10 μℓ of the chloroform layer were blotted on filter paper. The paper was sniffed for odor after evaporation of the solvent.

EXAMPLE 1

A human malodor neutralization study was conducted to determine whether the combination of cupric sulfate and sodium bicarbonate exhibits deodorant efficacy 5 minutes, 3 hours, 6 hours, and 24 hours after application to the axilla. Three separate panels of 8-12 men, each of whom were known to be either low, medium or high odor producers, were evaluated. They abstained from the use of all underarm products throughout the study. On Day 0 the axilla which had the stronger malodor for each subject was determined. This axilla was then treated on Days 1 and 2 with 0.5mℓ of 2 wt/vol% aqueous cupric sulfate followed 5 minutes later with 10 wt/vol% aqueous sodium bicarbonate. The other axilla was untreated. Judges evaluated each panelist's axillae 5 minutes, 3 hours, 6 hours, and 24 hours after treatment. The axillae were washed between Day 1 and Day 2.

The untreated underarms showed an increase in odor levels throughout the test period after the first post-control "sniff", while the treated underarms showed an apparent decrease in odor after the treatment and no increase in odor for at least six hours. The data was statistically analyzed and the decrease was found to be statistically significant for the high odor producers five minutes after the sodium bicarbonate treatment, and at three hours and six hours thereafter; and statistically significant for the medium odor producers five minutes after the sodium bicarbonate treatment, and at three hours, six hours, and twenty-four hours. The low odor panel showed lower odor production on the treated side at three and six hours, but the difference was not statistically significant.

Product types suitable for application to the axilla to neutralize malodor include aqueous alcoholic solutions, lotions, creams, ointments, powders, suspensions, soaps, waxes, gels, stick forms and compositions for pressurized dispensing in the form of an aerosol. The following examples are exemplary of such product types.

EXAMPLE 2

|  | Wt. % |
|---|---|
| **Aerosol:** | |
| Cupric Sulfate | 1.0 |
| Sodium Bicarbonate | 5.0 |
| Isopropyl Myristate | 3.7 |
| Fumed Silica | 0.15 |
| Perfume | 0.25 |
| Propellants | q.s. to 100 |

EXAMPLE 3

**Stick:**

| | |
|---|---|
| Volatile Silicone 7158 (Union Carbide) | 46 |
| Cupric Sulfate | 3 |
| Sodium Bicarbonate | 17 |
| Stearyl Alcohol | 24 |
| Polyethylene Glycol Distearate 6000 | 6 |
| Carbowax PEG 1540 (Union Carbide) | 4 |

EXAMPLE 4

**Dry Stick:**

**Powder Phase**

| | |
|---|---|
| Avicel PH-105 (FMC) | 52.35 |
| Italian Talc | 14.30 |
| Cupric Sulfate | 3.00 |
| Sodium Bicarbonate | 16.00 |
| Dri-flo Starch 4951 (National Starch) | 7.30 |
| Zinc Stearate | 1.90 |

**Liquid Phase**

| | |
|---|---|
| Volatile Silicone 7207 (Union Carbide) | 4.80 |
| Isopropylan 33 (Robinson-Wagner) | 0.10 |
| Perfume | 0.25 |

EXAMPLE 5

**Roll-On:**

| | |
|---|---|
| Magnesium Aluminum Silicate | 1.0 |
| Deionized Water | 58.7 |
| Glyceryl Monostearate | 8.0 |
| Cupric Sulfate | 5.0 |
| Sodium Bicarbonate | 25.0 |
| Volatile Silicone | 2.0 |
| Perfume | 0.3 |

FABRIC TREATMENT

EXAMPLE 6

70% cotton/30% polyester blend dress shirts were selected for use in the test. Kodel IV 100% polyester (Test-fabrics Inc. number 965) patches (13" x 7") were sewn to the shirt underarms to determine the retention of malodor by the 100% polyester. The polyester fabric was not fabric finished for soil release, etc., and was washed as part of the garment assembly using industry standard washing procedures designed by the American Association of Colorists and Chemists (AATCC). An actual wear test was undertaken in which a reproducible, odor-producing panelist (wearing no antiperspirant or deodorant through the test protocol) wore all garments. In addition, an expert panel of malodor evaluators was used for malodor determination. The wearing and laundering cycle was repeated until sufficient malodor retention on the garments could be detected several hours after wear. No undershirts were worn under test garments. Although significant odor was developed after a wearing period of six hours, the odor was completely removed using a standard washing procedure for several wear-laundering cycles. After three or four cycles, however, noticeable malodor retention was detectable after laundering.

As part of the test, newly prepared shirts were worn for several days between laundering in hopes of producing a more severe malodor problem. After laundering, only slight malodor was determined by the panel of evaluators. The malodor appeared to be accumulative in the patches.

Unfinished 100% polyester shirts were laundered as received under standard conditions. These shirts were worn for four-hour periods, evaluated for malodor retention and laundered with the following ingredients:

26.7 g of AATCC Standard Detergent 124*

42 ℓ of water 48,9°C (120°F.)

840 g of cupric sulfate (anhydrous)

2.7 lb load of fabric

| *AATCC STANDARD DETERGENT 124 | |
|---|---|
| Nominal Composition | wt. % |
| Linear alkylate sulfonate - sodium salt | 14.0 |
| Alcohol ethoxyiate | 2.3 |
| Soap - high molecular weight | 2.5 |
| Sodium tripolyphosphate | 48.0 |
| Sodium silicate ($SiO_2/Na_2O$-2.0) | 9.7 |
| Sodium sulfate | 16.0 |
| CMC | 0.25 |
| Moisture and Miscellaneous** | 7.25 |
| | $\overline{100.00}$ |

**In the AATCC Standard Detergent 124 with optical brightener (fluorenscent whitening agent), miscellaneous includes approximately 0.3% of a mixed brightener system including balanced proportions of bis-(triazinyl)-stilbenedisulfonate and triazolylstilbenesulfonate.

The addition of sodium bicarbonate to the detergent plus cupric sulfate solution can be used to increase the solution pH from 4 to 8 to improve the cleaning efficiency of the detergent formulation. Cupric sulfate was the active ingredient added to the washing solution to eliminate malodor retention. Unfinished shirts were laundered with and without cupric sulfate.

Following drying and a cool-down cycle, the odor evaluating panel determined that the regular laundered shirt had odor retention whereas the shirt laundered with the formulation containing cupric sulfate showed no odor retention after laundering and drying.

Unfinished polyester, soil-release-finished polyester, and polyester/cotton blend shirts were evaluated in a wearing and regular laundering protocol without additives. The soil-release-finished shirt had a greater retention of malodor than did the unfinished polyester. The blend shirt malodor produced during wearing was almost completely removed by the regular laundering cycle.

## Claims

1. A method of neutralizing malodor derived from the axilla of a subject, characterized by contacting the malodor by topically applying to the axilla of the subject, or by applying to a fabric which was or will be in contact with said malodor, as a neutralizing compound cupric sulfate, in such a malodor - reducing concentration as to provide a cosmetic improvement of the subject by reduction or elimination of malodor associated with the subject.

2. The method of claim 1, characterized by applying sodium bicarbonate to the malodor before, concurrently with or after said contacting with said neutralizing compound.

3. The method of claim 1 or 2, characterized in that the neutralization occurs in the location of the axilla.

4. The method of any one of the preceding claims characterized in that the concentration of said neutralizing compound is from 0.001 to 100% by weight.

7

**5.** The method of any one of the preceding claims characterized in that the concentration of said neutralizing compound is from 0.05 to 5.0% by weight.

**6.** The method of any one of the preceding claims, characterized in that said contacting is with aqueous cupric sulfate at about 2% wt/vol.

**7.** The method of any one of claims 2 to 6, characterized in that the contacting with sodium bicarbonate is at a concentration from 0.01% to 99.9% by weight.

**8.** The method of any one of claims 2 to 6, characterized in that the contacting with sodium bicarbonate is at a concentration from 0.1% to 10% by weight.

**Patentansprüche**

**1.** Verfahren zum Neutralisieren eines von der Axilla einer Person stammenden, überriechenden Stoffes, dadurch gekennzeichnet, daß Kupfer(II)sulfat als Neutralisiermittel in einer solchen geruchsmindernden Konzentration mit dem überriechenden Stoff in Berührung gebracht wird, daß der üble Geruch der Person vermindert oder beseitigt wird, und daß dazu das Neutralisiermittel topisch auf die Axilla der Person oder auf einen Textilstoff aufgetragen wird, der mit dem überriechenden Stoff in Berührung gestanden hat oder in Berührung kommt.

**2.** Verfahren nach Anspruch 1, dadurch gekennzeichnet, daß vor dem oder gleichzeitig mit dem oder nach dem Inberührungbringen des Neutralisiermittels mit dem überriechenden Stoff Natriumbicarbonat aufgetragen wird.

**3.** Verfahren nach Anspruch 1 oder 2, dadurch gekennzeichnet, daß das Neutralisieren an der Axilla vorgenommen wird.

**4.** Verfahren nach einem der vorhergehenden Ansprüche, dadurch gekennzeichnet, daß das Neutralisiermittel in einer Konzentration von 0,001 bis 100 Gew.-% verwendet wird.

**5.** Verfahren nach einem der vorhergehenden Ansprüche, dadurch gekennzeichnet, daß das Neutralisiermittel in einer Konzentration von 0,05 bis 5,0 Gew.-% verwendet wird.

**6.** Verfahren nach einem der vorhergehenden Ansprüche, dadurch gekennzeichnet, daß der überriechende Stoff mit wäßrigem Kupfer(II)sulfat bei einer Konzentration von etwa 2 Gew./Vol.-% in Berührung gebracht wird.

**7.** Verfahren nach einem der Ansprüche 2 bis 6, dadurch gekennzeichnet, daß der überriechende Stoff mit Natriumbicarbonat in einer Konzentration von 0,01 bis 99,9 Gew.-% in Berührung gebracht wird.

**8.** Verfahren nach einem der Ansprüche 2 bis 6, dadurch gekennzeichnet, daß der überriechende Stoff mit Natriumbicarbonat in einer Konzentration von 0,1 bis 10 Gew.-% in Berührung gebracht wird.

**Revendications**

**1.** Procédé pour neutraliser une mauvaise odeur provenant de l'aisselle d'un individu, caractérisé par la mise en contact de la mauvaise odeur avec du sulfate de cuivre en tant que composé neutralisant par application topique à l'aisselle de l'individu ou par application à un tissu qui a été ou qui sera en contact avec ladite mauvaise odeur, en une concentration réduisant la mauvaise odeur telle que l'on obtient une amélioration cosmétique de l'individu par réduction ou élimination de la mauvaise odeur associée à l'individu.

**2.** Procédé selon la revendication 1, caractérisé par l'application de bicarbonate de sodium à la mauvaise odeur, avant, en même temps que ou après ladite mise en contact avec ledit composé neutralisant.

**3.** Procédé selon la revendication 1 ou 2, caractérisé en ce que la neutralisation se produit au niveau de l'aisselle.

4.  Procédé selon l'une quelconque des revendications précédentes, caractérisé en ce que la concentration dudit composé neutralisant est de 0,001 à 100 % en poids.

5.  Procédé selon l'une quelconque des revendications précédentes, caractérisé en ce que la concentration dudit composé neutralisant est de 0,05 à 5,0 % en poids.

6.  Procédé selon l'une quelconque des revendications précédentes, caractérisé en ce que ladite mise en contact est accomplie avec du sulfate de cuivre aqueux à environ 2 % en poids/volume.

7.  Procédé selon l'une quelconque des revendications 2 à 6, caractérisé en ce que la mise en contact avec le bicarbonate de sodium est accomplie à une concentration de 0,01 % à 99,9 % en poids.

8.  Procédé selon l'une quelconque des revendications 2 à 6, caractérisé en ce que la mise en contact avec le bicarbonate de sodium est accomplie à une concentration de 0,1 % à 10 % en poids.